# EUROPEAN PATENT SPECIFICATION

(11) **EP 1 536 768 B1**
(45) Date of publication and mention of the grant of the patent: **08.07.2009**
(21) Application number: 03792102.0
(22) Date of filing: 15.08.2003
(51) Int. Cl.: A61K 9/00, A61K 47/34

(54) **BIODEGRADABLE COMPOSITION WITH PROLONGED RELEASE OF AN ANTITUMOUR AGENT AND PREPARATION THEREOF**
BIOLOGISCH ABBAUBARE ZUSAMMENSETZUNG MIT VERLÄNGERTER FREISETZUNG EINES ANTITUMORMITTELS UND IHRE HERSTELLUNG
COMPOSITION BIODEGRADABLE A LIBERATION PROLONGEE D'AGENTS ANTITUMORAUX ET MODE DE PREPARATION

(30) Priority: 22.08.2002 CZ 20022860
(43) Date of publication of application: 08.06.2005
(73) Proprietor: Pliva-Lachema A.S., 621 33 Brno (CZ)
(72) Inventor: DITTRICH, Milan, 500 11 Hradec Králové (CZ); SOVA, Petr, 500 02 Hradec Králové (CZ)
(74) Representative: Pavlica, Tomas
(86) International application number: PCT/CZ2003/000046
(87) International publication number: WO 2004/017937

(56) References cited:
- EP-A- 0 290 983
- SK-B- 279 067
- US-A- 5 143 730
- US-A- 5 783 205
- US-B1- 6 420 454
- HAMPL, JAROSLAV ET AL: "Adjuvant activity of linear aliphatic polyester and branched aliphatic oligoester microspheres" INTERNATIONAL JOURNAL OF PHARMACEUTICS (1996), 144(1), 107-114 , XP002263886

## Description

### Field of invention

The invention relates to a biodegradable composition with prolonged release of an antitumour agent and the preparation thereof.

### Background of invention

High-molecular compounds based on polymers and copolymers of glycolic and lactic acids, possessing linear constitution of chains, have been used since the 1960's for production of surgical sutures and/or orthopedic joining devices [Middleton, J. C. , Tipton, A. J., Synthetic biodegradable polymers as orthopedic devices, Biomaterials 21 (2000) 2335-2346]. DL-lactic acid/glycolic acid copolymers with higher ratios of lactic acid or with equimolar ratio of both monomers, have been extensively studied and commercially exploited for formulation of biodegradable implantable systems of particulate type [Arshady, R. (ed) : Microspheres, Microcapsules & Liposomes (1. ed. ) vol. 2, pp. 1291, Citus, London 1999] or monolithic type [Rothen-Weinhold, A. et al., Injection-molding versus extrusion as manufacturing technique for the preparation of biodegradable implants, Eur. J. Pharm. Biopharm. 48 (1999) 113-121]. In most cases these implants or microparticles contain peptides with gonadotrophic activity. Information has been published about the advantageous release profiles of bromocriptin mesylate from the microparticular systems based on the microspheres prepared from the high-molecular copolymers of glucose with lactic acid, synthesised by the ring- opening polymerisation [Kissel, T. et al. , Parenteral depot-systems on the basis of biodegradable polyesters, J. Contr. Rel. 16 (1991) 27- 42].

A series of patents by Atrix Laboratories uses the principle of application of the solution of a biocompatible polymer as the minor component in a mixture with a biocompatible water-miscible solvent as the markedly major component. After the application of the solution by syringe, the solvent is rapidly distributed into the adjacent muscle or other soft tissues and the in situ implant is formed [Dunn, R. L. et al., Biodegradable in-situ forming implants and methods of producing the same, US-Patent 4,938, .763 ; Shively, M. L. et al., Physico-chemical characterization of a polymeric injectable implant delivery system, J. Contr. Rel. 33 (1995) 237-243; Kranz, H. et al., Myotoxicity studies of injectable biodegradable in-situ forming drug delivery systems, Int. J. Pharm. 212 (2001) 11-18 ; Coonts, B. A. et al., Biodegradation and biocompatibility of a guided tissue regeneration barrier membrane formed from a liquid polymer material, J. Biomed. Mater. Res. 42 (1998) 303-311]. This method of drug delivery has found a practical application in the pharmacotherapy (Yewey, G. et al., Liquid delivery compositions, PCTW095/27481). A similar method for the in situ implant formation was carried out using glycofurole as in the aqueous medium miscible solvent of lactic acid/glycolic acid copolymer [Eliaz, R. E. , Kost, J., Characterization of a polymeric PLGA-injectable implant delivery system for the controlled release of proteins, J. Biomed. Mater. Res. 50 (2000) 388-396]. Furthermore, a subcutaneous application of a gel by syringe with subsequent photopolymerisation of reagents applied and the cross-linking of the polymeric chains has been described [Elisseeff, J. et al. , Transdermal photopolymerization for minimally invasive implantation, Proc. Natl. Acad. Sci. USA 96 (1999) 3104-3107]. Injectable protein compositions, based on the phase transitions of aqueous collagen solution at temperatures below 45 °C have been patented [Jones, R. E. , Li, M. T. , Novel collagen formulations, PCTW096/33696].
Polyesters with branched chains have a higher random coil density compared to polyesters with a linear chain constitution. This is connected with their smaller volume and lower viscosity of their melts and solutions [Colby, R. H. et al., Scaling properties of branched polyesters, 2. Static scaling above the gel point, Macromolecules 25 (1992) 7180-7187; Gorda, K. R., Piffer, D. G. , Star-shaped condensation polymers: Synthesis, characterization, and blend properties, J. Appl. Polym. Sci. 50 (1993) 1977-1983]. The above mentioned properties are very advantageous for the carrier processing. They are preferably synthesized with ring opening [Kim, S. H. et al. , Preparation of star-shaped polylactide with pentaerythritol and stannous octoate, Macromol. Chem. 194 (1993) 3229-3236]. Using polycondensation of polycarboxylic acids or polycarboxylic hydroxysubstituted acids with glycolic acid, the slightly cross-linked oligoesters, insoluble in water and soluble in dimethyl formamide, were prepared [Wada, R. , Hyon, S. H. , Ikada, Y., New biodegradable oligoesters for pharmaceutical application, J. Biomater. Sci. Polymer Edn. 7 (1996) 715-725]. By branching of the structure, a more convenient course of degradation, swelling and erosion can be achieved, which can positively influence the continuity of release kinetics of active compounds of the low- molecular and high-molecular types [Breitenbach, A., Li, Y. X, Kissel, T. , Branched biodegradable polyesters for parenteral drug delivery systems, J. Contr. Rel. 64 (2000) 168-178]. Synthesis of star-shaped block c.opolymers of caprolactam polyols with a polyactide has been patented. Non-crystalline products with a low glass transition temperature is suitable for production of packages and for other applications (Ford, T. M. , Easily degradable star-block copolymers, US Patent 5,399, 666). Composition of matrices based on branched polyester carriers, prepared by very slow polycondensation of citric acid with propylene glycol, exhibits a continuous release of biological active compounds (Lindahl, A. , Hagslatt, H. , Bryland, R. , Biologically active composition, PCTW099/58108). Synthesis based on the copolymerisation under the ring opening of lactides and multifunctional alcohols or sugars led to formation of high molecular star-shaped copolymers suitable for preparation of bio-compatible foams (Spinu, M. , Ford, T. M. , Degradable foam materials, US Patent 5, 210, 108).

### Summary of invention

The subject of the invention is a biodegradable antitumour composition with prolonged release of an antitumour agent destined for the administration into tissues, where the composition comprises at least one antitumour agent and a carrier, cinsisting of biodegradable oligoester having the numeric mean relative molecular weight Mₙ from 650 to 7 500, the mass mean relative molecular mass M_{w} from 800 to 10 000 and glass transition temperature Tg from-35 to 45 °C, and which is prepared by polycondensation reaction of polyhydric alcohol containing at least 3 hydroxy groups with at least one aliphatic α--hydroxy acid in the molar ratio of polyhydric alcohol to aliphatic α-hydroxy acid being from 0.5:99.5 to 12.0:88.0, wherein the central molecule of biodegradable oligoester is a polyhydric alcohol, to the hydroxy groups of which chains created from several molecules of at least one aliphatic α--hydroxy acid are bound by ester bonds, and wherein it is in the form of homogeneous one-phase solution, micellar colloid system, one-phase or two-phase gel, suspension, paste oe emulsion.

The biodegradable antitumour composition advantageously further comprises at least one liquid biocompatible plasticizer, wherein the weight ratio of at least one biocompatible plasticizer to biodegradable oligoester is from 1:20 to 9:10.

Advantageously, the liquid biocompatible plasticizer is soluble in the carrier and imperfectly soluble or insoluble in water.

The biodegradable antitumour composition advantageously further comprises at least one agent influencing the kinetics of the release of the antitumour agent.

The biodegradable antitumour composition advantageously further comprises at least one stabilizer of the antitumour agent or carrier.

The subject of the invention also is the preparation of the above-definer antitumour composition, wherein an antitumour agent, a carrier, and optionally a liquid biocompatible plasticizer, an agent influencing the konetics of the release of the antitumour agent, a stabilizer of the antitumour agent or a stabilizer of the carrier are heated to the temperature of 35 to 75 °C and mixed

The invention relates to constitution of compositions processed into the drug form of implants. The compositions are composed of two components, a carrier and an antitumour agent. The carrier is of the oligoester type, characterised by the branched structure of the molecule formed by a central molecule of polyhydric alcohol with three or more hydroxyl groups, to which oligoester branches represented by chains of aliphatic alfa-hydroxy acids, such as lactic acid, glycolic acid, hydroxybutyric acid, hydroxyvaleric acid, hydroxycapronic acid and optionally further homologs of alfa- hydroxyacids are connected. The systems exhibit plastic viscous behaviour, fractions with lowest molecular weights play the role of plasticizers in polydisperse mixture of oligoester carrier, improving the processability and thus enabling the application. The plastic behaviour of the oligoester biodegradable carriers can be advantageously emphasized by other additives, such as liquid biocompatible plasticizers. As optional additives, release modifiers of the antitumour agents or stabilisers can be also added.

These systems are preferably applied directly to the target tissue, using a syringe with a needle or other suitable devices. It is more convenient to increase the temperature of plastic systems by heating them before the application. The low level of swelling and the surface erosion of oligoester carrier are prerequisites for the surprisingly continuous and extended release of the antitumour agents with the possible consequence of enhanced biological activity.

The invention involves composition of pharmaceutical biodegradable preparations from oligoester carrier in the mixture with an antitumour agent with particular properties concerning the prolongation of release of the antitumout agent. The preparation with the composition according to this procedure is intended for the parenteral application and leads to an enhanced effect of the antitumour agents. The mixture of compounds constituting the drug form, implantable directly into the target tissue, consists of a biodegradable carrier, an antitumour agent or several such antitumour agents, and optionally a liquid biocompatible plasticizer, and also optionally other compounds, such as release kinetics modifiers of the antitumour agents or physical stabilizers of the overall structure and texture of the system, optionally also chemical stabilizers of individual components of the preparation according to the invention.

The biodegradable carrier is a low-molecular oligoester with a highly branched chain which is synthesized by polycondensation reaction. Multifunctional branching component is a polyhydric alcohol or sugar with three or more hydroxyl groups, such as glycerol, pentaerythritol, inositol, xylitol, mannitol, sorbitol, erythrose, threose, arabinose, ribose, gulose, idose, altrose, alose, talose, sorbose, mannose, glucose, fructose, galactose, sucrose, lactose. The chains of alfa-hydroxy acids, such as e. g. DL-lactic acid, glykolic acid, alfa-hydroxybutyric acid, alfa-hydroxyvaleric acid, alfa- hydroxy-capronic acid, are bonded by ester bonds to the molecule of polyhydric alcohol. The oligoester therefore possesses a star-shaped structure with a central molecule of polyhydric alcohol, which serves as a branching component during the polycondensation reaction. From this molecule, chains of different length are extending, while their maximum number is given by the overall number of hydroxyl groups of polyhydric alcohol or sugar.

The carrier is synthesized by polycondensation reaction of polyhydric alcohol or sugar with alfa-hydroxy acid or with a mixture of several alfa-hydroxy acids in a molar ratio of alcohol to alfa- hydroxy acids ranging from 0.5 : 99.5 to 12: 88. The increase of the polyhydric alcohol content in the reaction mixture leads to a higher level of branching and to the limited growth of the chain. The reaction is carried out in a temperature interval from 120 to 220 °C.

The rate of reaction and the maximum level of conversion achieved, expressed as a parameter of number mean molecular weight Mn, increase as the pressure decreases. The presence of a catalyst, such as for example, acidic katex, phosphoric acid or toluene sulphonic acid, increases the reaction rate and also the maximum conversion ratio achieved. The Mn value of these size polydisperse materials ranges from 650 to 7500 and the corresponding Mw value is within the interval 800-10000; as measured by the GPC method, using the calibration for linear polystyrene molecules as standards. The level of polydispersity MW/Mn ranges from 1.2 to 12.0, usually being about 2.0. Lower-molecular fraction of oligoester carrier functions in the compositions according to the invention as a plasticizer lowering the glass transition temperature and also the viscosity, and hence, enables better processability and applicability. The products of polycondensation reaction can be used without purification or they can be purified by precipitation from the solutions in solvents possessing the relative permitivity £r from 4.0 to 32.0, by adding some quantity of water and subsequent drying of coacervate or precipitate. It is advantageous to use nonpurified reaction products, and hence to preserve their plasticized character.

Oligoester carriers obtained by the polycondensation reaction are colourless, yellow or brown semi-crystalline or amorphous materials with their glass transition temperatures Tg ranging from-35 °C to 45 °C. Due to the different reactivity and the different sterical hindrance of hydroxy groups towards the esterification reaction with carboxyl, conformationally flexible oligoester chains of various length can be formed on them. This structure is consequently characterised by surprisingly strong surface activity. The random coil of these branched molecules exhibits higher density and smaller volume. As a consequence, the viscosity of their melts and their concentrated solutions is lower, compared to linear oligoesters. The melts of branched oligoester or polyester carriers, possessing the above described structure, are as biomaterials serving as drug carriers more easily processed compared to oligoesters having linear chains constitution. Another advantage in this aspect is their easier applicability in drug compositions. When satisfactorily low viscosity is achieved, it enables to apply plastic systems, formed by carriers of this type, directly to the soft tissue by a syringe or by analogous suitable devices. This results in the formation of a definite irregularly shaped bolus, which fulfils the generally known and accepted definitions of medicinal implants.

Another substantiated advantage of the branched type of oligoesters is their low swelling. Surprisingly low swelling was found in case of carriers with a high branching level and very small molecular weight. The hydrolytic degradation of the branched chain carriers has a specific course with pronounced aspects of heterogeneous type. The hydrolysis proceeds via the mechanism of a gradual diminishing of the body. Contrary to the typical heterogeneous erosion from the surface of hydrophobic material, the erosion of oligoester particles proceeds via the mechanism of diffusion and elution of low-molecular, water-soluble degradation products into the biological hydrophilic surroundings. The body consisting of carriers of this type is in the aqueous environment or inside the organism gradually diminishing till it completely and without residues disappears. The oligoesters with linear chains, such as commercially available lactic acid/glycolic acid copolymer, degrade within the whole body, ie. homogeneously, this entirely different mechanism is given by a strong swelling of the body with continuous degradation within its whole volume, subsequent disintegration of the body into fragments and gradual dissolution and disappearance of these fragments. The hydrolysis of ester bonds in polyesters with linear chains happens randomly, no specific site with preferential cleavage within the linear chains has been found. The branched structures used in the invention have not been studied in this aspect.

The low level of swelling and the above-described heterogeneous nature of the degradation of branched carriers create conditions for continuous long-term release of the antitumour agents into the surrounding target tissues. Biodegradable materials of branched, star-shaped oligoester types are demonstrably biocompatible as carriers for the antitumour agents. They are hydrolytically cleaved by water in the biological environment to yield non-toxic compounds soluble in water, which are eliminated or metabolised.

Among the antitumour agents, suitable for the application in the invention, it is possible to mention pharmaceuticals from the group of folic acid derivatives, such as metothrexate etc. , pyrimidine analogues, such as 5-fluorouracile etc. , the group of alkylating agents, such as cisplatinum and derivatives thereof, complexes of platinum in oxidation state IV, the group of urea derivatives, such as carmustine (BCNU), lomustine (CCNU) etc. , the group of antibiotics with an intercalation mechanism, such as antibiotics from the group of anthracycline antibiotics, such as doxorubicine, idarubicine etc. , derivatives of anthracene, and anthrapyrazoles, such as mitoxantrone, oracine etc., the group of mitosis inhibitors such as vinca alkaloids, and also taxanes such as paclitaxel, docetaxel, the group of topoisomerase inhibitors, such as topotecane, captotecine etc. , etopozide, tenipozide etc. , while well applicable are also compounds from the group of hormones such as gonadoliberine analogues, gosereline, leuproline etc. , group of CDK inhibitors (cycline dependant kinases), preferably trisubstituted and tetrasubstituted.

The compositions can also contain compounds influencing immunity, such as for example cyclosporine, muramyldipeptide, muramyltripeptide, tetanus toxoid, diphteria toxoid, interpherones, interleukines, cytokines, enterotoxines, viral and bacterial antigens, vaccines, immunogenic adjuvants for vaccination.

Another optional ingredient of the compositions according to the invention is a liquid plasticizer, advantageously used for better application of implantable mixtures. For mixtures according to the invention, the especially preferred ones are liquid plasticizers molecularly miscible with the carrier, such as citric acid triesters, e. g. triethyl citrate, tributyl citrate, trihexyl citrate and mixture thereof, mono-and diesters of citric and tartaric acids, lactic acid esters, such as ethyl lactate, butyl lactate, octyl lactate, dodecyl, lactate, tetradecyl lactate, hexadecyl lactale and their mixtures, glycerol esters, such as triacetine, tributyrine, tricapryline, alkylesters of benzoic acid, such as C1o-13- or C1215-alkylesters, furthermore also dibutyl adipate, dioctylcyclohexane, octyl dekanoate, cetyl stearyl octanoate etc. Plasticizers and mixture of the above mentioned and other plasticizers are characterised by molecular miscibility with oligoester carriers, however, also by a limited or very limited miscibility with water, they can even be virtually immiscible with water. They are used as minor ingredients of ternary mixtures of carrier, active compounds and optional components in concentrations ranging from 5 to 45% of weight. Liquid plasticizers as ingredients of the compositions according to the invention can be used individually or in mixtures.

As optional ingredients improving the properties of biodegradable and biocompatible implant-forming compositions according to the invention modifiers of the release of the antitumour agents can be used. These adjuvants can operate in particular in interaction with oligoester carrier by the mechanism of hydrofilisation of the mixture. Inorganic and organic salts, sugars, urea, tenzides can be used. As agents influencing the carrier degradation by changing pH in compositions according to the invention, various acids, aminoacids, amines, salts, oxides and hydroxides can be used. Biocompatible and biodegradable adjuvants in the mixtures according to the invention can influence the kinetics and the level of swelling and the rate of carrier degradation. As pharmaceutical stabilizers various compounds, used as cryoprotectants can be used, as well as hydrophilic colloids, preservatives, antimicrobial agents, antioxidants, sequestrants, osmotic agents etc.

Biodegradable implants according to this invention are prepared by mixing of weighted components, that is oligoester biodegradable carrier, antitumour agents and optional ingredients, such as plasticizers, modifiers of pharmaceutics release or stabilizers. The mixture is heated at a temperature ranging from 35 to 95 °C, preferably at 40 to 75 OC and more preferably at 45 to 60 °C. Thereafter it is agitated in a suitable apparatus. This highly viscous plastic mixture can be filled in a suitable hermetically sealed vessel or to the cylinder of a syringe. The mixtures can be stored in a well sealed vessel or in a syringe of a suitable construction made from a suitable material and equipped with a suitable cap, placed in a covering protecting the mixture from air humidity and light.

The application of plastic compositions according to the invention, i. e. biodegradable carrier, antitumour agent and optional plasticizer, modifier of release or stabilizer, can be done by a syringe with tubing, trocar needle or needle of a suitable size. More viscous mixtures with higher glass transition temperatures Tg or with higher concentrations of active compounds can be heated before the application. When the composition according to the invention is applied to a tumour, it is possible to make use of the fact known from the literature dealing with physiology, concerning the temperature limit of contact compatibility. This limit is 52 °C in humans.

The advantage of the implant according to the invention is achieving better compliance in patients. The patient is less psychically and physically stressed after application of the compositions according to the invention, with several days, several weeks or several months lasting release and effect of the active compounds than in case of frequently repeated applications of antitumour agents in a classical pharmaceutical form, such as injectable solution or suspension for injections. The release period of biologically active compound from the composition according to the invention ranges from several days to several months. This period can be influenced by the carrier type, type of structural units from reactive monomers, molecular weight of biodegradable carrier and the level of branching of the molecule. Another factor of prolongation of antitumoue agent release are physico-chemical properties of the pharmaceutical and its concentration in the implant. It has been found that the plasticizers according to this invention have surprisingly low influence on the kinetics of release of many antitumour agents while considerably lowering the viscosity of implantable compositions. The release can be controlled by modifiers, i. e. additives influencing the osmotic, acidity and other aspects of the systems according to this invention. The local release of often very toxic antitumour agents ensures their high concentrations in the target tissue at low levels in the circulatory system. This leads to a substantially lower incidence of undesirable side effects, i. e. toxicity, mutagenity, teratogenicity, imunogenity etc. By application of implants with compositions according to the invention with continuous long-lasting input of the antitumour agent to the corresponding site, not only quantitatively, but also qualitatively novel and distinct effects in the preventive, palliative, causal or radio-sensibillised acting of antitumour agents can be achieved.

### Examples

### Example 1

2000.0 mg of sorbitol/DL-lactic acid copolymer synthesised by polycondensation of both ingredients in a molar ratio 1:22, having the values Mₙ = 1350, M_{w} = 2150, T_{g} = -9 °C was mixed with 100.0 mg of paclitaxel in a beaker under aseptic conditions. The mixture was heated to 50 °C and well mixed. 200.0 mg of the resulting mixture was weighed out into vials with perforable caps. The vials were closed and sterilised by gamma-radiations with overall dose of 25 kGy.

### Example 2

25.5 g of pentaerythritol/DL-lactic acid/glykolic acid terpolymer, prepared by polycondensation in the molar ratio 1:12:12 having the values Mₙ = 2200, M_{w} = 4500, T_{g} = 7,5 °C was mixed with 10.0 g of tributyl citrate and 5.5 g cisplatinum in a beaker. The mixture was heated to 70 °C and well mixed. 1.0 g aliquots of the resulting suspension were filled into syringes with 2 ml nominal volume. The syringes were closed by caps instead of needles and a plunger was set on. Everything together with a desiccant was hermetically wrapped into a foil laminated with an aluminum layer. The wrapped syringes were sterilised by the overall dose of gamma-radiations of 25 kGy and they were placed into a refrigerator.

### Example 3

Irregular particles of approximately hemispherical shapes were formed by extrusion of the mixture from Example 2 at the bottom of scintillation vials. Two vials were filled by the samples weighing m = 150 mg, another two vials were filled by samples of m = 450 mg weight. The samples were overlaid with 15.0 ml of TRIS-buffer 0.05 mol.l⁻¹ isotonic sodium chloride pH 6.0 and they were placed in a thermoregulated shaker with water bath at a temperature of 37 °C. The same series of sample was prepared with buffer at pH 7.0. Samples of the dissolution liquid were collected at the time intervals of 1, 3, 5, 7, 10, 14, 19, 26 and 35 days and they were analysed for the content of cisplatinum by the AAS method. After each sampling, the dissolution medium was entirely changed for a fresh one. The results of dissolution tests are stated in the following Table 1.

**Table 1**

| **m (mg)** | **pH** | **X** | **1** | **3** | **5** | **7** | **10** | **14** | **19** | **26** | **35** |
|---|---|---|---|---|---|---|---|---|---|---|---|
| 150 | 6.0 | X₁ | 11.6 | 21.5 | 32.7 | 47.2 | 62.8 | 87.9 | 96.9 | 104.2 | 104.7 |
| | | X₂ | 14.3 | 23.0 | 35.6 | 50.4 | 64.9 | 91.5 | 99.4 | 101.6 | 103.1 |
| | 7.0 | X₁ | 16.7 | 24.8 | 35.2 | 48.6 | 64.8 | 84.1 | 93.2 | 94.0 | 96.6 |
| | | X₂ | 17.7 | 28.4 | 39.3 | 54.9 | 64.0 | 87.5 | 96.8 | 100.5 | 100.9 |
| 450 | 6.0 | X₁ | 0.9 | 8.7 | 23.8 | 43.6 | 52.7 | 68.1 | 83.5 | 93.2 | 95.6 |
| | | X₂ | 6.5 | 11.3 | 27.0 | 37.2 | 47.9 | 63.8 | 84.8 | 94.9 | 95.9 |
| | 7.0 | X₁ | 5.4 | 12.6 | 31.7 | 43.8 | 58.4 | 70.3 | 75.8 | 83.0 | 89.6 |
| | | X₂ | 6.2 | 11.2 | 31.4 | 47.2 | 62.6 | 82.7 | 83.3 | 94.2 | 96.3 |

Based on the results stated in Table 1, it can be concluded that the release of cisplatinum is not dependent on the actual acidity of the system. The rate of release of the cisplatinum decreases with the increase of the implant size.

### Example 4

Metothrexate (2.50 g) was mixed with 2.50 g poloxamer 407 and 20.0 g DL-lactic acid/glykolic acid/mannitol terpolymer to yield the sample 4A. The terpolymer was synthesized in molar ratios of the reactants 5: 47.5:47.5 and had the values Mₙ = 2650, M_{w} = 5800 and T_{g} = 19.1.

Binary mixtures containing 10% of metothrexate and 90% of terpolymer (sample 4B), and further ternary mixture of 10% metothrexate, 30% tributyrine and 60% terpolymer (sample 4C) and also quaternary mixture of 10% metothrexate, 30% tributyrine, 0.5% zinc stearate and 59.5% terpolymer (sample 4D) were prepared analogously.

The mixtures were heated at 70 °C and mixed in a ultrasonic field of Ikasonic 50 U probe. The mixtures were filled at 2.00g +/- 0.10 g aliquots into syringes. The syringes were set on by a plunger and wrapped into a foil laminated with an aluminum layer. Wrapped syringes were sterilised by the overall dose of gamma-radiations 25 kGy and stored in refrigerator at 3 °C.

### Example 5

150 mg of implantable mixtures from Example 4 were placed to the bottom of scintillation vials. Two vials with weighed mixtures from each of the experiments using 4A, 4B, 4C and 4D mixtures were prepared. The mixtures were overlaid with isotonic phosphate-citrate buffer at pH 7.0 and the vials were tightly closed by screwing cap. Then they were placed in a shaking thermostat tempered at 37 °C. The samples were collected at the intervals of 1 day, 3 days, 7 days, 14 days, 21 days and 28 days, and measured on spectrophotometer at λ = 283 nm. The results of liberation tests are stated in the following Table 2, demonstrating release of metothrexate from the matrices according to Examples 4 and 5.

**Table 2**

| Time (days) | 4A | 4B | 4C | 4D |
|---|---|---|---|---|
| 1 | 14,6 | 2,5 | 2,1 | 2,9 |
| | 25,7 | 2,9 | 3;0 | 1,5 |
| 3 | 30,5 | 7,4 | 13,8 | 7,6 |
| | 39,8 | 8,2 | 13,5 | 5,3 |
| 7 | 45,3 | 12,4 | 18,4 | 11,8 |
| | 53,2 | 10,6 | 27,4 | 12,0 |
| 14 | 57,9 | 40,7 | 55,3 | 33,7 |
| | 62,1 | 38,2 | 50,1 | 45,8 |
| 21 | 63,0 | S 1,4 | 60,4 | 67,6 |
| | 64,6 | 58,3 | 66,7 | 68,8 |
| 28 | 65,7 | 74,6 | 70,9 | 101,1 |
| | 65,9 | 77,0 | 76,2 | 95,3 |

It is clear from the results of Table 2 that the release of metothrexate is long-term and continuous. The system is flexible. The required rate of liberation can be controlled by suitable additive and by its optimised concentration.

### Example 6

This example demonstrates the growth of mouse plasmacytome ADJ/PC6 in inbred mice BALN/c. The anti-tumour effect of biodegradable injectable composition according to Example 2 was tested by its intra-tumoral administration to inbred mice BALB/c.

In this experiments, inbred mouse of the strain BALB/c, females, their body mass ranging from 10 to 20 g, were used, and they were s.c. transplanted a mouse plasmacytome of the line ADJ/PC6 to the right hip. When tumours achieved the size of approximately 1 cm³, a single dose applied 0.1 ml of the formulation preheated at 45 to 50 °C, containing 0.0 mg CDDP/kg and 16.0 mg CDDP/kg or 35.5 mg CDDP/kg was applied intratumorally.

The animals were divided into four groups, each comprising six animals: 1. tumours with no application, i.e. the control group, 2. group with tumours and i.t. applied carrier alone, i.e. placebo group, 3. i.t. applied 16.0 mg of CDDP/kg, 4. i.t. applied 35.5 mg of CDDP/kg.

During the experiments the tumours were measured and the weights of mice were determined (every 3. or 4. days) and the tumour volumes were calculated using the equation 1 x w x π/6. The day 35 after the transplantation, i.e. day 20 after application of the composition, the mice were put to death, the tumours were weighed and the % tumour growth inhibition (%TGI) and tumour weight inhibition (%TWI) were calculated. (%TGI) = (1-(average volume of tumour in treated group/average volume of tumour in control group) x 100; (%TWI) = (1-(average weight of tumour in treated group /average weight of control group) x 100. The tumour growth inhibition (percentage) and tumour weight inhibition (percentage) on day 35 after the start of experiment are stated in the following Table 3.

**Table 3**

| Group | CDDP dose | %TGI | %TWI |
|---|---|---|---|
| 1. Control | - | 0 | 0 |
| 2. Placebo | - | 5.16 | 4.46 |
| 3. | 16.0 mg / kg | 99.81 | 98.66 |
| 4. | 35.5 mg / kg | 99.84 | 99.84 |

## Claims

1. Biodegradable antitumour composition with prolonged release of an antitumour agent destined for the administration into tissues, **characterised in that** it comprises at least one antitumour agent and a carrier, consisting of biodegradable oligoester, having the numeric mean relative molecular mass Mₙ from 650 to 7 500, the mass mean relative molecular mass M_{w} from 800 to 10 000 and the glass transition temperature T_{g} from -35 to 45 °C, and which is prepared by polycondensation reaction of polyhydric alcohol containing at least 3 hydroxy groups with at least one aliphatic α -hydroxy acid in the molar ratio of polyhydric alcohol to aliphatic α -hydroxy acid being from 0.5:99.5 to 12:88, wherein the central molecule of biodegradable oligoester is a polyhydric alcohol, to the hydroxy groups of which chains created from several molecules of at least one aliphatic α -hydroxy acid are bound by ester bonds, and **in that** it is in the form of homogenous one-phase solution, micellar colloid system, one-phase or two-phase gel, suspension, paste or emulsion, and wherein the weight ratio of anti-tumour agent to carrier is from 1:1 to 1:100 000.

2. The composition according to claim 1, **characterised in that** it further comprises at least one liquid biocompatible plasticizer, wherein the weight ratio of at least one biocompatible plasticizer to biodegradable oligoester is from 1:20 to 9:10.

3. The composition according to claim 2, **characterised in that** the liquid biocompatible plasticizer is soluble in the carrier and imperfectly soluble or insoluble in water.

4. The composition according to claims 1 to 3, **characterised in that** it further comprises at least one agent influencing the kinetics of the release of the antitumour agent.

5. The composition according to claim 1 to 4, **characterised in that** it further comprises at least one stabilizer of the antitumour agent or carrier.

6. The preparation of the antitumour composition according to claims 1 to 5, **characterised in that** an antitumour agent, a carrier, and optionally a liquid biocompatible plasticizer, an agent influencing the kinetics of the release of the antitumour agent, a stabilizer of the antitumour agent or a stabilizer of the carrier are heated to the temperature of 35 to 75 °C and mixed.

## Patentansprüche

1. Biologisch abbaubare Anti-Tumor-Komposition mit verlängerter Freisetzung eines Anti-Tumor-Mittels, das für Dosierung ins Gewebe bestimmt ist, **dadurch gekennzeichnet, dass** es mindestens ein Anti-Tumor-Mittel und einen Träger beinhaltet, der aus einem biologisch abbaubaren Oligoester besteht, eine numerisch mittlere relative Molekülmasse Mₙ von 650 bis 7 500, eine massen-mittlere relative Molekülmasse M_{w} von 800 bis 10 000 und eine Glasübergangstemperatur T_{g} -35 bis 45°C hat und der durch eine Polykondensationsreaktion eines polyhydrischen Alkohols gewonnen wurde, welcher mindestens 3 Hydroxy-Grruppen mit mindestens einer aliphatischen Alfa-Hydroxysäure in molarem Verhältnis des polyhydrischen Alkohols zu den aliphatischen Alfa-Tlydroxysäuren von 0,5:99,5 bis 12:88 umfasst, wobei die zentrale Moleküle des biologisch abbaubaren Oligoesters der polyhydrische Alkohol ist, an dessen Hydroxy-Gruppen durch Ester-Bindungen aus mehreren Molekülen mindestens einer aliphatischen Alfa-Hydroxysäure gebildete Ketten angebunden sind, und dass sie die Form einer homogenen Einphasen-Solution, eines mizellaren Kolloid-Systems, eines Einphasen- oder Zweiphasengels, einer Suspension, einer Paste oder einer Emulsion hat, wobei das Verhältnis des Anti-Tumor-Mittels zu dem Träger von 1:1 bis 1:100 000 ist.

2. Die Komposition nach Anspruch 1, **dadurch gekennzeichnet, dass** sie weiter mindestens einen flüssigen biokompatiblen Weichmacher umfasst, wobei das Verhältnis des mindestens einen biokompatiblen Weichmachers zu dem biologisch abbaubaren Oligoester zwischen 1:20 bis 9:10 ist.

3. Die Komposition nach Anspruch 2, **dadurch gekennzeichnet, dass** der flüssige biokompatible Weichmacher in dem Träger löslich und im Wasser unvollkommen löslich ist.

4. Die Komposition nach Ansprüchen 1 bis 3, **dadurch gekennzeichnet, dass** sie weiter mindestens ein Mittel beinhaltet, das die Kinetik der Freisetzung von dem Anti-Tumor-Mittel beeinflusst.

5. Die Komposition nach Anspruch 1 bis 4, **dadurch gekennzeichnet, dass** sie weiter mindestens einen Stabilisator des Anti-Tumor-Mittels oder des Trägers umfasst.

6. Vorbereitung der Anti-Tumor-Komposition nach Ansprüchen 1 bis 5, **dadurch gekennzeichnet, dass** ein Anti-Tumor-Mittel, ein Träger und eventuell ein flüssiger biokompatibler Weichmacher, ein die Kinetik der Freisetzung des Anti-Tumor-Mittels beeinflussende Mittel und ein Stabilisator des Trägers erwärmt an die Temperatur von 35 bis 75°C und vermischt werden.

## Revendications

1. Une composition biodégradable antitumorale à une libération prolongée d'un principe actif antitumoral destinée pour l'administration à un tissu, **caractérisée en ce qu'**elle comprend au moins un principe actif antitumoral et un support consistant en oligoester biodegradable ayant une moyenne masse moléculaire relative numérique Mₙ comprise entre 650 et 7 500, une moyenne masse moléculaire relative pondérale M_{w} comprise entre 800 et 10 000 et une température de transition vitreuse T_{g} comprise entre -35°C et 45°C, en ce que'elle est préparée par une réaction de polycondésation d'un alcool polyhydrique contenant au moins 3 groupes hydroxy avec au moins un α-hydroxyacide aliphatique utilisés dans un rapport molaire du alcool polyhydrique à l'α-hydroxyacide aliphatique compris entre 0,5:99,5 et 12:88, la molécule centrale du oligoester biodegradable étant formée par l'alcool polyhydrique aux groupes hydroxy duquel chaînes composées de plusieurs molécules d'au moins un α-hydroxyacide aliphatique sont liées à l'intermédiaire de liaisons ester, et **en ce qu'**elle est dans une forme d'une solution homogène à une phase, d'un système colloïdal micelaire, d'un gel à une ou deux phases, d'une suspension, d'une pâte ou d'une émulsion, the rapport pondéral du principe actif antitumoral au support étant compris 1:1 et 1:100 000.

2. La composition selon la revendication 1, **caractérisée en ce qu'**elle en plus comprend au moins un agent plastifiant liquide biocompatible, the rapport d'au moins un agent plastifiant liquide biocompatible à l'oligoester biodegradable étant compris entre 1:20 et 9:10.

3. La composition selon la revendication 2, **caractérisée en ce que** l'agent plastifiant liquide biocompatible est soluble dans le support et imparfaitement soluble ou insoluble dans l'eau.

4. La composition selon les revendications 1 à 3, **caractérisée en ce qu'**elle en plus comprend au moins un agent influençant la cinétique de la libération du principe actif antitumoral.

5. La composition selon les revendications 1 à 4, **caractérisée en ce qu'**elle en plus comprend au moins un agent stabilisant le principe actif antitumoral ou le support.

6. Une préparation de la composition antitumoral selon les revendications 1 à 5, **caractérisée en ce que** l'on chauffe le principe actif antitumoral, le support et éventuellement l'agent plastifiant liquide biocompatible, l'agent influençant la cinétique de la libération du principe actif antitumoral, l'agent stabilisant le principe actif antitumoral ou l'agent stabilisant le support à une température comprise entre 35°C et 75°C et l'on mélange lesdits constituants.
